Europäisches Patentamt

European Patent Office

Office européen des brevets

(11) Publication number: **0 058 684**
**B1**

# (12) EUROPEAN PATENT SPECIFICATION

(45) Date of publication of patent specification: **21.05.86**

(21) Application number: **81902317.7**

(22) Date of filing: **13.08.81**

(86) International application number:
**PCT/BR81/00005**

(87) International publication number:
**WO 82/00662 04.03.82 Gazette 82/07**

(51) Int. Cl.⁴: **C 13 K 1/10,** C 08 B 30/00,
C 08 B 37/18, C 12 N 1/16,
C 12 P 7/06, C 12 P 7/08,
C 12 P 7/10, C 12 P 7/14,
C 12 P 19/14, C 12 P 19/20,
C 12 P 19/24

(54) **PROCESS FOR PRODUCING CARBOHYDRATES FROM VEGETABLE JUICE.**

(30) Priority: **27.08.80 BR 8005458**

(43) Date of publication of application:
**01.09.82 Bulletin 82/35**

(45) Publication of the grant of the patent:
**21.05.86 Bulletin 86/21**

(84) Designated Contracting States:
**AT CH DE FR GB LI LU NL SE**

(56) References cited:
**US-A-3 607 647**

**CHEMICAL ABSTRACTS, vol. 85, no. 2, July 12,
1976, page 121, abstract no. 7560w,
COLUMBUS OHIO (US)**

**CHEMICAL ABSTRACTS, vo. 71, no. 26,
Decembr 29, 1969, page 135, abstract no.
126263x, COLUMBUS OHIO (US)**

(73) Proprietor: **PELLEGRINI, Armando Paulo
Rua General Dionîsio Cerqueira 307
30000 Belo Horizonte Minas Gerais (BR)**

(72) Inventor: **PELLEGRINI, Armando Paulo
Rua General Dionîsio Cerqueira 307
30000 Belo Horizonte Minas Gerais (BR)**

(74) Representative: **Hirsch, Marc-Roger
34 rue de Bassano
F-75008 Paris (FR)**

Courier Press, Leamington Spa, England.

## Description

The present invention refers to a process for producing carbohydrates derived from vegetable juice.

In the field of production of carbohydrates from vegetable juice, no substantial innovation has been introduced in recent years, except for some mechanical improvements to devices used for such production. Thus, the methods currently used still are based on the crystallization of sucrose and the mere separation of the resulting solid phase from the liquid phase.

Carbohydrates are used on a large scale for various purposes throughout the world. The fact that by fermenting carbohydrates, alcohol is produced suffices to show their importance. By esterifying carbohydrates, biodegradable and thus non-polluting detergents are produced and used in various fields and in large quantities, thus holding an important place in modern technology.

By acid degradation of carbohydrates in addition to other compounds, furfural is produced; by alkalizing them lactic acid is obtained which has a wide range of uses, including the production of food, esters, plasticizers and plastics. By oxidation, allylation and a wide spectrum of hydrogenation reactions of carbohydrates, including numerous secondary reactions, food, resins, plastics, plasticizers, explosives, cosmetics, adhesives, and the like, are produced.

Currently, large scale production of carbohydrates from sugar cane, beets, sorghum or any syrup capable of producing crystalline carbohydrates, proceeds according to the following overall scheme: initially, the raw material is introduced into machines wherein the juice is mechanically extracted and collected. Thereafter, the impurities present in the juice are precipitated by chemical and thermical treatment, after which the impurities are removed by conventional filtration procedures, the filtrate then being subjected to vacuum concentrating and crystallization operations, and finally to separation of the resulting solid and liquid phases.

Practically all the methods of producing carbohydrates, including condensation carbohydrates having a high molecular weight, as well as those containing three, five or six carbon atoms, lead to obtaining the most conveniently usable soluble form, i.e. the a-delta-glucopyranose.

It is known that the types of carbohydrates most common in nature are cellulose and starch, the latter serving as reserve nourishment for plants and the former constituting their skeleton, both these carbohydrates being insoluble in water. With the exception of these two, the remaining carbohydrates occurring in nature form solutions, part of which can be transformed into solid matter, whereas another part, in spite of technological progress, often representing significant quantities will remain liquid.

From an industrial viewpoint, the existance of the two phases is disadvantageous because, in addition to the depletion of the desired solid phase, the liquid phase is subject to chemical and biological reactions which generally are not controllable.

US Patent Application No. 4 111 750 (Colilla et al.) discloses a process for converting liquefied starch into a mixture of glucose and fructose, wherein a multicomponent immobilized enzyme system is used which comprises immobilized glucoamylase, glucose isomerase and debranching enzyme such as immobilized pullulamase, immobilized isoamylase or a mixture thereof. In addition to the fact that this prior publication is not particularly concerned with the problem of obtaining the desired carbohydrates in the form of solids, the known process is related only to the conversion of starch, but not to the treatment of any convenient vegetable juice.

Chemical Abstracts, vol. 71, 1969, item 126263x describes a procedure of reducing the viscosity of beet sugar juice by hydrolyzing the pectin with a pectolytic enzyme (Pectinax), and thus does not concern a process of the kind considered herein.

Chemical Abstracts, vol. 85, 1976, item 85:7560W reports the use of endogenous enzymes in the sugar manufacture, but does not disclose any specific method of preparing carbohydrates, substantially only in the solid state, from any convenient vegetable juice.

On the contrary, one major object of the present invention is to provide a process of preparing carbohydrates in the solid state from a vegetable juice.

Another object of the invention is to provide a process as defined hereinabove, which is adapted to be carried out easily and reliably at low cost, and which requires less equipment then comparable known processes do.

The present invention is aimed at overcoming the drawbacks of the known methods, and with this object in view provides a process for producing carbohydrates from vegetable juice, characterized by the steps of:

a.—extracting said vegetal juice from raw vegetal material;

b.—filtering said vegetal juice to remove material suspended therein;

c.—subjecting said vegetal juice resulting from step b.— to an enzymatic reaction at a pH ranging from 3.5 to 7 at a temperature ranging from 18 to 67°C for a period of time ranging from 20 to 90 minutes, by successively:

i) admixing said vegetal juice in the first stage with 3.2.1.4.-b-1,4-glucan glucanhydrolase in an amount of 1.5U and 3.2.1.15-poly-a-1,4-galacturonic glucan hydrolase in an amount of 0.9U;

ii) admixing said vegetal juice in the second stage with 3.2.1.20-a-D-glucoside glucohydrolase in an amount of 0.3U;

iii) admixing said vegetal juice in the third stage with 3.2.1.21-b-D-glucoside glucohydrolase in an amount of 0.3U; 5.3.1.4-L-arabinose-ketol-isomerase in an amount of 0.15U and 5.3.1.5-D-xylose-ketol-isomerase in an amount of 0.15U; and

iv) admixing said vegetal juice in the fourth stage with 3.2.1.1-a-1,4-glucan-4-glucanhydrolase in an amount of 0.12U and 3.2.1.3-a-1,4-glucan glucohydrolase in an amount of 0.22U, the unit U being the amount of each of the said enzymes required to hydrolyse one mole of starch in 10 minutes,

d.—filtering and/or centrifuging the vegetal juice resulting from the enzymatic reaction of step c.— to produce a carbohydrate containing cleaned phase;

e.—evaporating under reduced pressure said cleaned phase to remove water therefrom; and

f.—crystallizing the product resulting from step e.— thereby producing said carbohydrates in solid form.

Preferably, after completion of the enzymatic reaction of step c.— and prior to filtering and/or centrifuging the vegetal juice in step d.—, said vegetal juice is heated to a temperature ranging from 90 to 100°C so as to clot any impurities present therein.

In a preferred embodiment of the invention the above-mentioned cleaned phase is evaporated under reduced pressure in step e.— to remove water therefrom at a temperature of up to 70°C.

When carrying out the process according to the invention, the vegetable raw material used is preferably constituted by sugar cane, beets, sorghum or any syrup capable of producing crystalline carbohydrates.

The process of the present invention thus relates to the production of carbohydrates comprising the implementation of specific controlled enzymatic reactions on previously determined components of the juice, while the remaining components do not react thereby finally producing the solid phase only. With this process of the invention, by the judicious utilization of adequate hydrolases and of transferases of the enzymes classification, it is possible to obtain completely crystallizable and soluble carbohydrates that can be stored over long periods of time without decomposition, these carbohydrates being adapted to be subjected to reactions such as hydrogenation, esterification, oxidation and the like.

In the process of the present invention, the utilization of enzymes reacting in successive steps provides the total trasnformation of carbohydrates into carbohydrates having six to twelve carbon atoms, for direct use or for subsequent applications, stemming from a primary product and obtained exclusively in solid phase. In this process, the mere action of hydrolase-oxireductases-transferases on the juice leads to specific and final results that even nature does not always manage to achieve.

For a better understamding, the carbohydrates produced by the conventional process and by the process according to the present invention, respectively, are listed in qualitative terms hereinbelow:

| Conventional process | | Enzymatic process according to the present invention | |
|---|---|---|---|
| Sucrose | (solid and liquid) | | |
| Sucrose<br>Fructose } | Main Carbohydrates (Liquid) | Sucrose<br>Glucose } | Main Carbohydrates (Solids) |
| Galactose<br>Xylose<br>Ramnose<br>Arabinose<br>Pectins<br>Starch<br>Uronic Acid<br>Methoxyl<br>Gums<br>Colloids<br>Dextrines<br>D-manitol } | Secondary Carbohydrates (Liquid) | | |
| Aconitic Acid<br>Inosite<br>Phytin } | Other Carbohydrates (Liquid) | Aconitic Acid<br>Inosite<br>Phytin } | Other Carbohydrates (Liquid) |
| Basic Ashes<br>Acid Ashes<br>Proteins<br>Aminoacids } | Liquid | Basic Ashes<br>Acid Ashes<br>Proteins<br>Aminoacids } | Solids |
| Esteroids<br>and Waxes } | Liquid | Esteroids<br>and Waxes } | Solid |
| Antocyanins<br>Chlorophyll<br>Polyphenols<br>Tannin } | Pigments (Liquid) | Pigments (Traces) } | Solid |
| Other nitrogenous compounds<br>Vitamins<br>B Complex<br>Water } | Liquid | Vitamins<br>B Complex<br>Other nitrogenous compounds<br>no nitrogenous acid } | Solid |

From the comparison of both types of products, it is clear that the product of the enzymatic process presents extremely important aspects. Because of the initial treatment of the juice, the resulting carbohydrates when incorporated into a final product improve the latter's nourishment characteristics because of the carbohydrates greater nutritive value, since the carbohydrates such as gums, colloids, xylose, arabinoses, starch, dextrines, pectins, ramnose and galactose, are converted into glucose. Moroever the carbohydrates produced in accordance with the present invention are important in the production of industrial alcohol, since the carbohydrates contain 90—92% of TRS as compared to the TRS content of 55—60% of the juice used in conventional processes, and all of these are fermentable, thereby providing, under well controlled conditions, an ethanol output of up to 90%.

The carbohydrates of the present invention are defined as being those produced by an enzymatic process wherein the end product is constituted by a single solid phase. By acting on the extract under controlled conditions, the reaction dynamics are so induced as to be capable of completing nature's actions. By acting on previously determined substances, the process according to the present invention produces a specific, well defined product that basically retains all the initial components, since only reactions that were interrupted in the so called "relative vegetal cycle" are completed, in which the juice acts as a natural and primary vehicle.

Other objects, features and advantages of the present invention will become apparent from the following detailed description which refers to the appended Figures and is given by way of illustration, but not of limitation.

Figure 1 is a simplified flow chart of the basic steps of a currently used known industrial process; and

Figure 2 is a simplified flow chart of the basic steps of the process according to the present invention.

As shown in Figure 1, the juice is extracted from the vegetable matter by a conventional extracting device 1 and introduced into filter means 2 which can be provided with a wire grid so as to remove gross material in suspension in the extracted juice. The juice is then heated by heater means 3 where the juice is cleaned by heat, chemical coagulation and even ionic exchange. Plate heaters 4 are provided to increase the temperature of the extracted juice. A vacuum concentrator battery 5 is provided wherein most of the water is eliminated whereafter the material is passed to solidifiers 6 and then to centrifugal separators 7, where solids are finally separated.

Figure 2 illustrates the process of the present invention wherein some of the heaters 3, the centrifugal separators 7 and some of the plate heaters 4 are eliminated, whereas other conventional components amongst those mentioned hereinabove are retained, and wherein an enzymatic reaction unit or reactor 8 not shown in the drawing is added.

The process of the present invention is carried out in the following manner. By means of the extractor 1, the juice is extracted from the vegetable material, such extracted juice containing carbohydrates dissolved therein. There is no need to control the pH, since the vegetable extracts are in the 3.5—7 range, in which the proposed enzymes are active. The reaction times in reactor 8 vary from 20 to 90 min. The temperature should be maintained at a value comprised between 18—67°C. The enzymes utilized during the different reaction stages performed in enzyme reactor 8 are listed below, by the number corresponding to their international classification, and in the present case they vary from 3.2.1.1 (4-glucan-4-glucohydrolase) to 5.3.1.5 (D-xylose-ketol-isomerase).

| Name | Dosage (quantity) |
|---|---|
| 1st stage with addition of: | |
| 3.2.1.4-b-1,4-glucan glucanhydrolase | 1.5U |
| 3.2.1.15-poly-a-galacturonic glucanhydrolase | 0.9U |
| 2nd stage with addition of: | |
| 3.2.1.20-a-D-glucoside glucohydrolase | 0.3U |
| 3rd stage with addition of: | |
| 3.2.1.21-b-D-glucoside glucohydrolase | 0.3U |
| 5.3.1.4-L-arabinose-ketol-isomerase | 0.15U |
| 5.3.1.5-D-xylose-ketol-isomerase | 0.15U |
| 4th stage with addition of: | |
| 3.2.1.1-a-1,4-glucan-4-glucanhydrolase | 0.12U |
| 3.2.1.3-a-1,4-glucan-glucohydrolase | 0.22U |

The "U" unit-based dosage of the enzymes is equivalent to the amount capable of hydrolyzing one mol of starch in 10 min.

After the enzymatic reaction stages are completed, the temperature of the thus extracted material is preferably raised to 90—100°C by means of heaters 3 so as to clot all impurities that may still be present, as well as the albuminoids. The material is then passed to filtration and/or centrifugation means so as to provide a cleaned phase of about 13 Brix. The thus cleaned juice is then sent to vacuum concentrators 5 to eliminate practically all water, the concentrators being preferably maintained at a temperature not exceeding 70°C and yielding a juice of about 70° Brix. Once adequate saturation is reached so that the juice forms crystals, the product is stirred at low speed in the crystallizers 6 until the granules separate by themselves.

The end product must have a relative humidity of less than 0.5%, preferably 0.25%. The resulting concentrate is composed of carbohydrates, no liquid phase being present. The process of the present invention, provides, as an option, that the concentrates is dried by convenient means to form an even powder or granulate mass which can be used directly or in further industrial operations of the types previously mentioned herein.

Amongst the more significant advantages obtained by using the process of the present invention the following may be cited:

—no losses occur in the primary process;

—one single solid phase is present in the resulting primary product;

—a strictly natural product composition is obtained;

—absence of effluents and by-products;

—about 30% increase of the end production;

5

—greater ease of storage and transportation;
—lower production cost since only small quantities of enzymes are used;
—elimination of part of the conventional equipment currently used;
—simplification of the production process, resulting in a decrease of cost; and
—obtention of an integral solid phase of natural components, without depletion due to phase distribution.

The following Table compares the conventional process and the enzymatic reaction process of the present invention.

| Conventional | Enzymatic |
|---|---|
| a) Extraction | a) Extraction |
| b) Chemical precipitation | b) Filtration |
| c) Heating (coagulation) | c) Enzymatic treatment |
| d) Filtration | d) Filtration and/or centrifugation |
| e) Evaporation | e) Evaporation |
| f) Cooking | f) Crystallization |
| g) Crystallization | |
| h) Centrifugation (triple) Sugar (sucrose) Syrup (discard) | |

It will be noted that in the enzymatic process of the present invention the operation for producing a dry crystalline product in the form of a single solid phase terminates in the crystallizer. In the conventional process this stage is constituted by the obtention of the so called "masse cuite" (baked paste) which is a mixture of crystals and honey, which mixture must be separated, otherwise both constituents will be lost by fermentation and other reactions. Consequently, when carrying out the enzymatic process according to the present invention, the carbohydrates and all other solid components present in the initial juice are recovered in the end product; no secondary carbohydrates, such as gums, colloids, xylose, arabinoses, starch or dextrins are present, which normally determine the high viscosity of juice. To decrease this viscosity the use of surfactants or detergents is required. In the process of the present invention all secondary carbohydrates are converted into glucose; the proportion of darkening agents and pigments like antocyanides and polyphenols, which are undesirable because of their color and taste is decreased; operational costs and equipment requirements are reduced; final revenues are increased; and natural carbohydrates are produced without solid and liquid phase distribution, which carbohydrates are perfectly adapted to be subjected to further uses and treatments.

It has been found that by conveniently adapting the process to the desired end product a large number of compounds could be converted into glucose, and that from an inactive form they were transformed into the most active carbohydrates, d-glucopyranose. Such, compounds were not destroyed, but recombined by the enzymes, so as to be converted into a more suitable and usable form. To obtain this, it has been found necessary to use the enzymes in four stages. Otherwise it was not found possible to obtain the end product resulting from carrying out the process in accordance with the present invention.

The carbohydrates thus obtained are sources of carbon which are useful for the production of other compounds through fermentation, i.e. due to the fact that they permit easy storage, transportation and handling.

Tests were undertaken with pure carbohydrates as obtained by the process of the present invention, as well as with such carbohydrates as enriched with salts. The results of these tests reveal that when using these carbohydrates high yields were achieved in the production of other compounds, for example yeasts and alcohol, in less time than when using carbohydrates produced by conventional processes.

The end product obtained by the current process is adapted to be used in a large range of applications, e.g. in the field of human nourishment, since it contains high amounts of sugars (around 92%), mineral salts, essential and non-essential amino-acids and vitamins. The product is also useful as nourishment for animals and for industrial purposes. It is efficiently used as raw material in alcoholic, glutanic and acetic fermentation processes and also in the production of a great variety of other products.

With regard especially to alcoholic fermentation, the results obtained using the whole sugar content of the product obtained by the process of the present invention for the production of ethanol have shown that such raw material was quite superior to conventional sugar.

It should be noticed, too, that the conventional process involves using molasses that contains non

6

fermentable carbohydrates whereas all the carbohydrates of the present invention are fermentable; and an 82% output is achieved without addition of nutrients, reaching 90 to 92% when nutrients are added, which represents better results than those of juice fermentation.

**Claims**

1. A process for producing carbohydrates from vegetal juice, characterized by the steps of:

a.—extracting said vegetal juice from raw vegetal material;

b.—filtering said vegetal juice to remove material suspended therein;

c.—subjecting said vegetal juice resulting from step b.— to an enzymatic reaction at a pH ranging from 3.5 to 7 at a temperature ranging from 18 to 67°C for a period of time ranging from 20 to 90 minutes, by successively:

i) admixing said vegetal juice in the first stage with 3.2.1.4.-b-1,4-glucan glucanhydrolase in an amount of 1.5U and 3.2.1.15-poly-a-1,4-galacturonic glucan hydrolase in an amount of 0.9U;

ii) admixing said vegetal juice in the second stage with 3.2.1.20-a-D-glucoside glucohydrolase in an amount of 0.3U;

iii) admixing said vegetal juice in the third stage with 3.2.1.21-b-D-glucoside glucohydrolase in an amount of 0.3U; 5.3.1.4-L-arabinose-ketol-isomerase in an amount of 0.15U and 5.3.1.5-D-xylose-ketol-isomerase in an amount of 0.15U; and

iv) admixing said vegetal juice in the fourth stage with 3.2.1.1-a-1,4-glucan-4-glucanhydrolase in an amount of 0.12U and 3.2.1.3-a-1,4-glucan glucohydrolase in an amount of 0.22U, the unit U being the amount of each of the said enzymes required to hydrolyse one mole of starch in 10 minutes,

d.—filtering and/or centrifuging the vegetal juice resulting from the enzymatic reaction of step c.— to produce a carbohydrate containing cleaned phase;

e.—evaporating under reduced pressure said cleaned phase to remove water therefrom; and

f.—crystallizing the product resulting from step e.— thereby producing said carbohydrates in solid form.

2. A process as claimed in claim 1, characterized in that subsequent to the enzymatic reaction of step c.— and prior to filtering and/or centrifuging the vegetal juice in step d.—, said vegetal juice is heated to a temperature ranging from 90 to 100°C so as to clot any impurities present therein.

3. A process as claimed in claim 1, characterized in that said cleaned phase is evaporated under reduced pressure in step e.— to remove water therefrom at a temperature of up to 70°C.

4. A process as claimed in claim 1, characterized in that said raw vegetal material is sugar cane, beets, sorghum or any syrup capable of producing crystalline carbohydrates.

**Patentansprüche**

1. Verfahren zur Herstellung von Kohlenwasserstoffen aus Pflanzensaft, dadurch gekennzeichnet, dass es folgende Schritte umfasst:

a.—Extrahieren des Pflanzensafts aus pflanzlichem Material;

b.—Filtrieren des Pflanzensafts zwecks Entfernung der darin suspendierten Stoffe;

c.—der im Verfahrensschritt b. erzielte Pflanzensaft wird während 20 bis 90 Minuten einer enzymatischenn Reaktion mit einem pH-Wert von 3,5 bis 7 und einer Temperatur von 18 bis 67°C mit folgenden nach einander erfolgenden Schritten unterzogen:

i) der Pflazensaft wird in einem ersten Schritt mit 1,50U 3.2.1.4-b-1,4-Glucan-Glucanhydrolase und 0,9U 3.2.1.15-poly-a-1,4-Galacturonglucanhydrolase gemischt;

ii) der Pflanzensaft wird in einem zweiten Schritt mit 0,3U 3.2.1.20-a-D-Glucosidglucohydrolase gemischt;

iii) der Pflanzensaft wird in einem dritten und Schritt mit 0,3U 3.2.1.21-b-D-Glucosidglucohydrolase und 0,15U 5.3.1.4-L-Arabinose-Ketolisomerase, sowie mit 0,15U 5.3.1.5-D-Xylose-Ketolisomerase gemischt;

iv) der Pflanzensaft wird sodann in einem vierten Schritt mit 0,12U 3.2.1.1-a-1,4-Glucan-4-Glucanhydrolase und mit 0,22U 3.2.1.-a-1,4-Glucan-Glucohydrolase gemischt, wobei die Einheit U diejenige Menge einer jeden der genannten Enzyme darstellt, die erforderlich ist, um ein Mol Stärke binnen 10 Minuten zu hydrolisieren,

d.—der bei der enzymatischen Reaktion im Schritt c. erzielte Pflanzensaft wird zwecks Erzeugung eines eine gerinigte Phase enthaltenden Kohlenwasserstoffs filtriert und/oder zentrifugiert;

e.—die gereinigte Phase wird zum Entfernen darin enthaltenen Wassers unter niedrigem Druck verdampft; und

f.—das im Schritt e. erzielte Produkt wird zwecks Herstellung der Kohlenwasserstoffe in fester Form kristallisiert.

2. Verfahren nach Anspruch 1, dadurch gekennzeichnet, dass nach der enzymatischen Reaktion des Schrittes c. und vor dem Filtrieren und/oder Zentrifugieren des Pflanzensafts im Schritt d. dieser Saft auf eine Temperatur von 90 bis 100°C erwärmt wird, um ggf. in ihm enthaltene Verunreinigungen zu agglomerieren.

**0 058 684**

3. Verfahren nach Anspruch 1, dadurch gekennzeichnet, dass die gereinigte Phase im Schritt e. under niedrigem Druck verdampft wird, um aus ihr bei einer Temperatur von bis zu 70°C Wasser zu entfernen.

4. Verfahren nach Anspruch 1, dadurch gekennzeichnet, dass das pflanzliche Material aus Zuckerrohr, Rüben, Sorghum oder einem zur Herstellung kristalliner Kohlenwasserstoffe geeigneten Sirup besteht.

**Revendications**

1. Un procédé de production d'hydrates de carbone à partir de jus végétal, caractérisé en ce qu'il comprend les étapes de:

a.—extraction des matières végétales brutes contenues dans ce jus végétal;

b.—filtration du jus végétal pour éliminer les matières en suspension qu'il contient;

c.—soumission du jus végétal résultant de l'étape b. à une réaction enzymatique à un pH compris entre 5 et 7 et à une température comprise entre 18 et 67°C pendant une période de temps comprise entre 20 et 90 minutes, par successivement:

i) dans la première étape, mélange du jus végétal avec une quantitié de 1,5U de 3,2,1,4-b-1,4-glucan glucanhydrolase et une quantité de 0,9U de glucan hydrolase de 3,2,1,15-poly-a-1,4-galacturonique;

ii) dans la deuxième étape, mélange du jus végétal avec une quantité de 0,3U de 3,2,1,20-a-D-glucoside glucohydrolase;

iii) dans la troisième étape, mélange du jus du végétal avec une quantité de 0,3U de 3,2,1,21-b-D-glucoside glucohydrolase; une quantité de 0,15U de 5,3,1,4-L-arabinose-cétol-isomérase et une quantité de 0,15U de 5,3,1,5-D-xylose-cétol-isomérase; et

iv) dans la quatrième étape, mélange du jus végétal avec une quantité de 0,12U de 3,2,1,1-a-1,4-glucan-4-glucanhydrolase et une quantité de 0,22U de 3,2,1,3-a-1,4-glucan glucohydrolase; l'unité U étant la quantité de chacune desdites enzymes requises pour hydrolyser une mole d'amidon en 10 minutes;

d.—filtration ou centrifugation du jus végétal résultant de la réaction enzymatique de l'étape c. pour produire une phase épurée contenant l'hydrate de carbone;

e.—évaporation sous préssion réduite de cette phase épurée pour éliminer l'eau qu'elle contient; et

f.—cristallisation du produit résultant de l'étape e. de façon à obtenir les hydrates de carbone sous forme solide.

2. Un procédé ainsi que revendiqué dans la revendication 1, caractérisé en ce que subsequemment à la réaction enzymatique de l'étape c. et préalablement à la filtration ou à la centrifugation du jus végétal de l'étape d., ce jus végétal est chauffé à une température comprise entre 90 et 100°C de façon à provoquer la coagulation de toutes impuretés qu'il contiendrait.

3. Un procédé ainsi que revendiqué dans la revendication 1, caractérisé en ce que la phase épurée est évaporée sous pression réduite dans l'étape e. afin d'éliminer l'eau qu'elle contient à une température pouvant atteindre 70°C.

4. Un procédé ainsi que revendiqué dans la revendication 1, caractérisé en ce que la matière végétale brute consiste en canne à sucre, betterave, sorgho ou tout sirop susceptible de produire des hydrates de carbone sous forme cristalline.